(19) Europäisches Patentamt
European Patent Office
Office européen des brevets

(11) Publication number: 0 080 275 A1

(12) # EUROPEAN PATENT APPLICATION

(21) Application number: 82305791.4

(22) Date of filing: 01.11.82

(51) Int. Cl.³: C 07 C 107/00, C 08 F 4/04

(30) Priority: 19.11.81 GB 8134919

(43) Date of publication of application: 01.06.83 Bulletin 83/22

(84) Designated Contracting States: BE DE FR GB IT NL

(71) Applicant: **IMPERIAL CHEMICAL INDUSTRIES PLC, Imperial Chemical House Millbank, London SW1P 3JF (GB)**

(72) Inventor: **Davies, Stephen Parry, 26 Clara Street South Yarra, Melbourne Victoria 3141 (AU)**
Inventor: **Thompson, Morice William, "Urishay" Highfield Lane Cox Green, Maidenhead Berkshire (GB)**

(74) Representative: **Wood, Dennis John Cecil et al, Imperial Chemical Industries PLC Legal Department: Patents Thames House North Millbank, London SW1P 4QG (GB)**

(54) **Preparation of azoiminoether hydrochlorides from azonitriles and their hydrolysis to azocarboxylic esters.**

(57) Azoiminoether hydrochlorides are prepared from azonitriles by a modification of the Pinner reaction in which the azonitrile is reacted under anhydrous conditions with a stoichiometrically equivalent amount of a monohydroxy compound in the presence of hydrogen chloride gas and of an ether group-containing compound.

EP 0 080 275 A1

ACTORUM AG

0080275

Pv. 32099/EP

## PREPARATION OF AZOIMINOETHER HYDROCHLORIDES FROM AZONITRILES AND THEIR HYDROLYSIS TO AZOCARBOXYLIC ESTERS

This invention relates to a process for the preparation of iminoether salts from certain azonitriles and monohydroxy compounds, and to the conversion of such salts to azo compounds which are useful as free radical-type initiators for the polymerisation of unsaturated monomers.

It is known that 2,2'-azobis(2-methylpropionitrile), for example, can be converted by reaction of the nitrile groups with a monohydroxy compound, in the presence of hydrogen chloride under anhydrous conditions, into the hydrochloride of the corresponding imino-ether:-

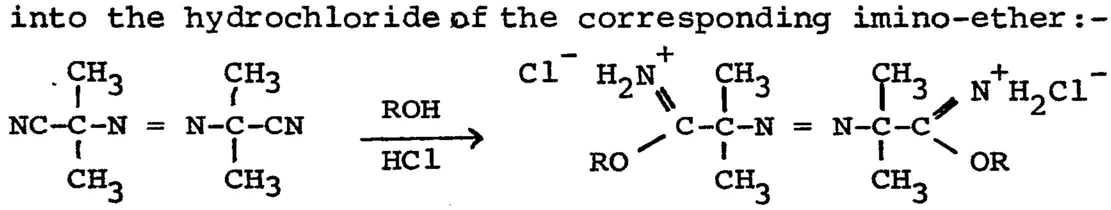


This conversion is based on the reaction first described by Pinner in Chem.Ber. 1883, 16, 352 and 1643, which is of fairly general application to nitriles. The imino-ether so obtained can be converted to other useful derivatives, eg, it can be hydrolysed to form the corresponding carboxylic ester, or it can be reacted with an amino-compound to give an amidine:-

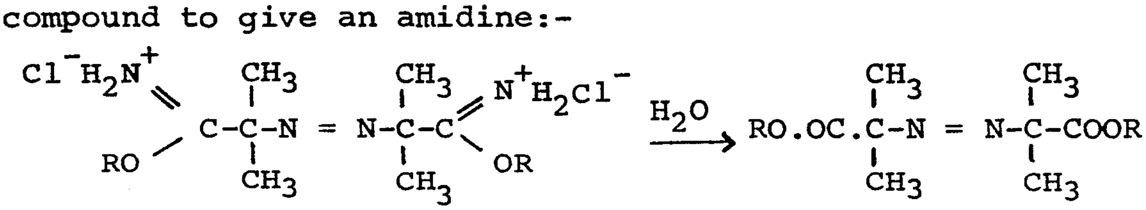


or

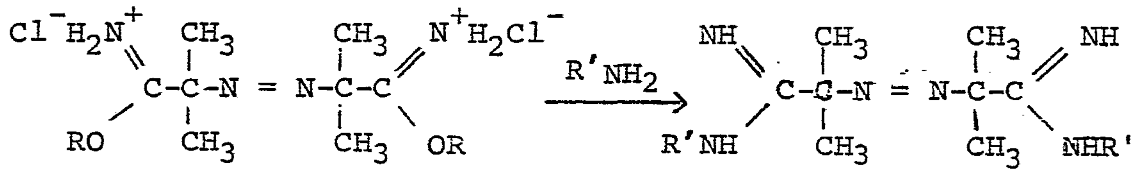

Both the above carboxylic ester and amidine derivatives of 2,2'-azobis(2-methylpropionic acid) are useful as free radical-type initiators in the polymerisation of ethylenically unsaturated monomers; the use of certain water-soluble amidines for this purpose has been described in European Patent Application No. 0009659A1.

In utilising the Pinner reaction in the above-described way, it has hitherto been found necessary to employ a stoichiometric excess of the monohydroxy-compound with which the azobisnitrile is reacted, in order to ensure that there is as nearly as possible complete conversion of the nitrile to the iminoether salt; this in turn requires that the iminoether salt be isolated free from excess alcohol before it can be further reacted as indicated above. In the case of some hydroxy-compounds, more particularly the lower alcohols, the iminoether hydrochloride does in fact precipitate out from the reaction mixture and its separation and purification is relatively straightforward. In other cases, however, including those where the hydroxy compound is of high molecular weight, the iminoether salt does not precipitate out and its isolation is then very troublesome. The need to use an excess of the hydroxy compound is also a disadvantage when that compound is expensive or not freely available.

We have now found that 2,2'-azobis(2-methylpropionitrile) and related compounds can be converted in excellent yield by means of the Pinner reaction to the coresponding iminoether hydrochlorides using only a stoichiometric proportion of the required monohydroxy compound,

if the reaction is carried out in the presence of a compound containing an ether grouping.

According to the present invention there is provided a process for the preparation of azoiminoether hydrochlorides of the general formula:-

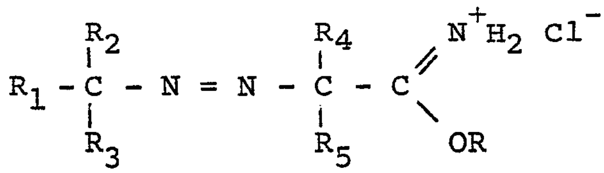

(I)

wherein R is the residue of a monohydroxy compound, $R_1$ is a methyl or ethyl group or a grouping of the formula -

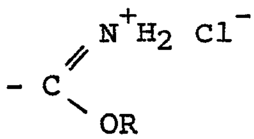

; $R_2$ is a methyl group or a β-carboxymethyl group and $R_3$ is a methyl group, or $R_2$ and $R_3$ together with the intermediate carbon atom form a cyclohexyl group; $R_4$ is a methyl group and $R_5$ is a methyl group, an ethyl group, a β-carboxyethyl group or a β-methoxy-ββ-dimethylethyl group, or $R_4$ and $R_5$ together with the intermediate carbon atom form a cyclohexyl group, the process comprising the reaction of an azonitrile of the formula

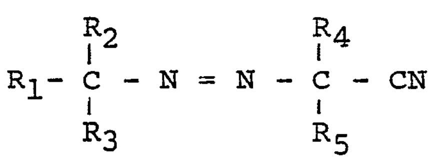

(II)

wherein $R_1$ is a methyl or ethyl group or a -CN group, $R_2$, $R_3$, $R_4$ and $R_5$ have the same significance as in formula (I), with a stoichiometrically equivalent amount of a monohydroxy compound R-OH in the presence of hydrogen chloride gas and of a compound containing in the molecule at least one ether grouping -C-O-C-, the reaction being conducted under anhydrous conditions.

Suitable azo nitriles which may be converted to the iminoether hydrochlorides by this process include 2,2'-azobis(2-methylpropionitrile), 2,2'- azobis(2-methylbutyronitrile), 4,4'-azobis(4-cyanopentanoic acid), 1,1'-azobis(1-cyanocyclohexane), 2-(2'-methylpropan-2'-azo)-2-cyano-4-methoxy-4-methylpentane, 1-(2'-methylpropan-2'-azo)-1-cyanocyclohexane, 1-(2'-methylbutan-2'-azo)-1-cyanocyclohexane, 2-(2'-methylpropan-2'-azo)-2-cyanopropane and 2-(2'-methylpropan-2'-azo)-2-cyanobutane.

Suitable monohydroxy compounds for use in the process include in particular the lower aliphatic monohydric alcohols such as methanol, ethanol and butanol, but higher members of the series such as hexanol or octanol may also be used.

Compounds containing in the molecule at least one ether grouping which are suitable for use in the process include diethylether, dioxan, 2-ethoxyethyl acetate and diethyleneglycol dimethylether, but in general any ether group-containing compound may be used provided that it is adequately soluble in the reaction mixture of the other constituents.

The proportion used of the ether group-containing compound is preferably sufficient to provide at least two ether oxygen atoms for each nitrile group present in the reaction mixture.

In addition to the azo nitrile, the monohydroxy compound and the ether group-containing compound, an essential ingredient of the reaction mixture is dry hydrogen chloride gas and this is preferably introduced by bubbling the gas through the mixture of the other reactants until the mixture is saturated, the temperature of the reaction mixture preferably being held below about 10°C by suitable cooling. When saturation has been achieved, the reaction mixture may be allowed to warm up to room temperature, at which the conversion of the nitrile

to the iminoether hydrochloride normally occurs satisfactorily over a period of some days. It is not normally desirable to carry out the conversion at temperatures much above ambient since the iminoether salt formed may then decompose to form the corresponding amide. If a faster conversion is desired, this may be achieved by increasing the concentration of hydrogen chloride in the reaction mixture by introducing it under pressure.

If desired, the reaction mixture may also contain a liquid diluent or solvent which is inert towards any of the other reactants and which preferably is sufficiently volatile to be removed, after the reaction is complete, by evaporation at a relatively low temperature (if necessary under reduced pressure) in order not to cause decomposition of the iminoether salt. Particularly suitable diluents include dichloromethane and chloroform; benzene and toluene may also be used, for example where complete removal of the diluent from the product is not required.

As already indicated, the process of the invention permits substantially quantitative conversion of the azonitrile to the iminoether hydrochloride. By contrast, if the azonitrile is reacted with a stoichiometrically equivalent amount of the monohydroxy compound in the absence of an ether group-containing compound, the conversion of the starting materials is far from complete even after prolonged contact and the product is consequently a complex mixture containing perhaps as little as 40% of the desired iminoether salt.

According to one particular embodiment of the invention, the monohydroxy compound and the ether group-containing compound may be one and the same, that is to say there may be used a single substance containing both

types of function in the molecule. This embodiment is of particular interest in the preparation of products having the above stated formula (I) wherein the group R is a high molecular weight residue from a poly(alkyleneoxy) derivative of a monohydric alcohol, more especially a water-soluble poly(ethyleneoxy) derivative. If a normal Pinner reaction is attempted in these circumstances, using a stoichiometric excess of the monohydroxypoly(alkyleneoxy) compound, it is found that the iminoether hydrochloride remains in solution and its separation from the unchanged hydroxy compound is a matter of considerable difficulty. If there is employed a stoichiometric equivalent amount according to the present invention, the reaction mixture contains essentially no unchanged starting materials and the question of separation of the product from then does not arise. Typical monohydroxy poly(alkyleneoxy) compounds include the monomethyl ethers of polyethylene glycols of molecular weight from 250 to 4000 and the monomethyl ethers of either random or block copolymers of ethylene oxide with another alkylene oxide such as propylene oxide or butylene oxide.

The substance containing both the hydroxy and the ether group function in the molecule is, however, not necessarily of high molecular weight and suitable low molecular weight substances of this type include, for example, 2-ethoxyethanol and the monoethyl ether of diethyleneglycol.

When the conversion of the nitrile to the iminoether is complete (this may most conveniently be recognised by the absence of the characteristic nitrile group peak at 2250 $cm^{-1}$, on infra-red spectroscopic examination of the reaction mixture) the iminoether hydrochloride may be

isolated by removing the liquid diluent and hydrogen chloride, preferably at about room temperature and, if necessary, under reduced pressure as already mentioned. Further purification of the imonoether salt may be carried out if required, but this is often a matter of some difficulty owing to the limited stability of this class of compound, and normally it is unnecessary where the compound in question is required only as an intermediate product which it is then desired to treat further, for example to hydrolyse to the corresponding carboxylic ester with the object of obtaining a final product which is useful as a free radical-type initiator for the polymerisation of unsaturated monomers. Thus, where hydrolysis of the imino-ether is intended, after removal of diluent and excess hydrogen chloride from the reaction mixture, the residue may simply be added to water. The resulting carboxylic ester may be recovered by filtration in suitable cases, or by extraction into a non-water-miscible solvent, such as dichloromethane, followed by evaporation of the solvent under reduced pressure. According to the nature of the group R as previously defined and the azonitrile employed, the azocarboxylic esters thus produced may be preferentially soluble in organic media and hence suitable for use as initiators in the polymerisation of unsaturated monomers either in bulk or in solution, or they may be preferentially soluble in aqueous media and hence useful in aqueous emulsion polymerisation processes. Certain of the esters are liquid at room temperature, which facilitates their handling in production-scale polymerisation processes.

The scope of the present invention is not intended to be limited by reference to any theoretical explanation of the manner in which it operates, but we believe that

the essential function of the ether grouping present in the reaction mixture, whether as a separate constituent or as part of the monohydroxy compound molecule, is to form a complex with the cation moiety of the iminoether hydrochloride, whereby the latter is stabilised and the progress of the conversion of the azonitrile towards completion is facilitated. We are aware of the paper by Walz, Bömer and Heitz published in Makromolekulare Chemie, 178, 2527-2534 (1977) in which there is described the synthesis of azo initiators by reacting together equimolar proportions of 2,2'-azobis(2-methylpropionitrile) and polyether diols in the presence of hydrogen chloride, but the resulting azo compounds are polymeric in nature and no mention is made of the possible significance of the presence of ether groupings in the diol either in the context of this particular synthesis or in any more general context.

The invention is illustrated but not limited by the following Examples, in which parts and percentages are by weight.

EXAMPLE 1

The monomethyl ether of polyethylene glycol of molecular weight 1950 (238g, 1 molar equivalent) was dried by azeotropic distillation with toluene and then dissolved in dichloromethane (300 ml.) 2,2'-Azobis(2-methylpropionitrile) (10.01 g, 0.5 molar equivalent) was added and dry hydrogen chloride gas was passed through the solution with cooling and stirring, keeping the temperature below about 10$^{o}$C. After about 8 hours, the solution had absorbed about 30 g of hydrogen chloride and was saturated. The reaction mixture was then allowed to stand at room temperature for 168 hours. Infra-red examination of the mixture at the end of this period indicated almost complete absence of the characteristic -C=N band at 2250 $cm^{-1}$. After the reaction mixture had stood at room temperature for a total of 264 hours, the solvents were removed under reduced pressure. The residue was poured into cold water (about 200 ml.) and extracted with dichloromethane (about 200 ml.) The organic phase was separated, dried with anhydrous magnesium sulphate and then concentrated to dryness under reduced pressure. The product was a waxy solid which was soluble in water. It was also soluble in methyl methacrylate but, in the presence of both water and methyl methacrylate, was preferentially soluble in the water phase. When examined by infra-red spectroscopy it exhibited a characteristic ester grouping peak at 1735 $cm^{-1}$; there was no peak observable at 2250 $cm^{-1}$, indicating the absence of nitrile groups. Thin-layer chromatography of the product and of the parent monomethyl ether of polyethylene glycol demonstrated that the two compounds were readily separated and distinguished; there was no evidence of contamination of the product with this starting material.

It was concluded therefore that the product was substantially the ester of polyethylene glycol(1950)monomethyl ether and 2,2'-azobis(2-methylpropanoic acid), as represented by the following formula:-

$$\left[ =N - \underset{\underset{CH_3}{|}}{\overset{\overset{CH_3}{|}}{C}} - COO(C_2H_4O)_{44}\ \ CH_2CH_2OCH_3 \right]_2$$

EXAMPLE 2.

The condensate of methanol (1 mol) with a mixture of ethylene oxide (24 mols) and propylene oxide (6 mols) (123 g, 0.07 mol.) was dried by azeotropic distillation with toluene and then dissolved in dichloromethane (300 g.) 2,2'-azobis(2-methylpropionitrile) (5.74 g, 0.035 mol) was added and dry hydrogen chloride gas was bubbled into the cooled and stirred reaction mixture, keeping the temperature below about 10°C. After about 15 g of hydrogen chloride had been passed, the solution was saturated. The mixture was then allowed to stand at room temperature for 5 days, following which the solvent and hydrogen chloride were removed under reduced pressure. The residue was stirred with water (200 ml) and extracted with dichloromethane. Removal of the solvent from the extract under pressure gave a colourless liquid which was insoluble in water. Examination of the product by infra-red spectroscopy indicated complete absence of nitrile groups; the product was concluded to be substantially the ester of the methanol/ethylene oxide/propylene oxide condensate and 2,2'-azobis(2-methylpropanoic acid), as represented by the formula:-

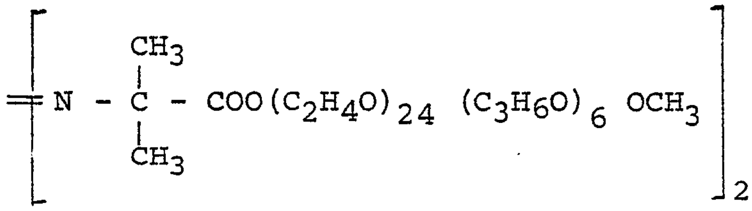

EXAMPLE 3

The monomethyl ether of polyethylene glycol of molecular weight 250 (50 g, 0.2 mol) was dried by azeotropic distillation with toluene and dissolved in dichloromethane (100 ml). 2,2'-Azobis(2-methylpropionitrile)(16.4 g, 0.1 mol) was added and dry hydrogen chloride gas was passed into the stirred and cooled solution until it was saturated, keeping the temperature below about 10°C. After the reaction mixture had stood at room temperature for 7 days, the solvent and hydrogen chloride were removed under reduced pressure. The residue was added to water (100 ml) and extracted with dichloromethane (150 ml). The extract was dried over anhydrous magnesium sulphate and then concentrated under reduced pressure to give a yellow liquid which was soluble in methyl methacrylate but only very slightly soluble in water. It was an efficient emulsifier for methyl methacrylate/water mixtures. Examination of the product by infra-red spectroscopy revealed a strong characteristic ester group absorption and absence of any nitrile group absorption. When subjected to thin-layer chromatography, the product exhibited a single streak which migrated differently from the monomethyl ether of the polyethylene glycol. The product was concluded to be substantially the ester of polyethylene glycol(250)monomethyl ether and 2,2'-azobis(2-methylpropanoic acid), as represented by the formula:-

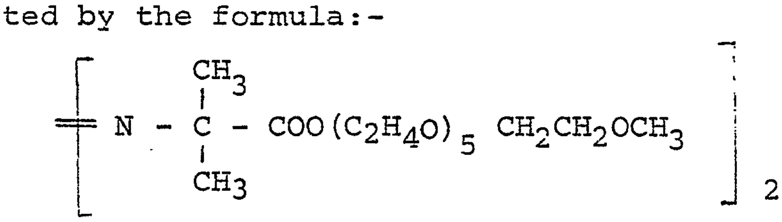

on the basis of which the yield of product isolated was about 75% of the theoretical amount.

EXAMPLE 4

Dry diethylene glycol monomethyl ether (52.2 g, 0.44 mol.) and 2,2'-azobis(2-methylpropionitrile)(36.1 g, 0.22 mol.) were together dissolved in dichloromethane (100 ml). Dry hydrogen chloride gas was bubbled through the cooled and stirred solution until it was saturated (about 15 g of hydrogen chloride had been passed at this stage) and keeping the temperature below about 10°C. The reaction mixture was allowed to stand at room temperature for 7 days and the solvent and hydrogen chloride was then removed under reduced pressure. The residue was poured into cold 10% aqueous potassium hydrogen carbonate (150 ml) and stirred. After 10 minutes, the mixture was extracted with dichloromethane (2 portions of 100 ml) and the extract concentrated under reduced pressure. The product was a yellow liquid which subsequently solidified to form needle-shaped colourless crystals. Infra-red spectroscopic examination of the product showed a strong ester group absorption and an absence of nitrile group absorption. The product was very slightly soluble in water, but soluble in methyl methacrylate. It was concluded to be substantially 2,2'-azobis [(3,6-oxaheptyl)2-methylpropanoate], having the formula:-

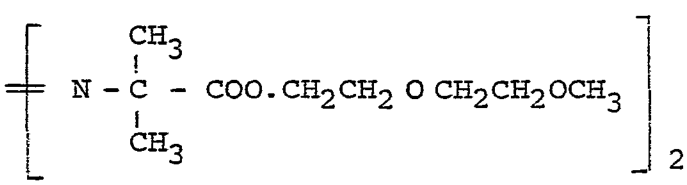

EXAMPLE 5

Dry 2-ethoxyethylacetate (18 g) was added to n-butanol (11.1 g, 0.15 mol) followed by 2,2'-azobis(2-methylpropionitrile)(12.3 g., 0.075 mol) and dichloromethane (85 g). Dry hydrogen chloride gas was bubbled through the solution with stirring and cooling. After about 7 g of hydrogen chloride had been passed, the solution was saturated and was then allowed to stand at room temperature for 6 days. The solvent and hydrogen chloride were removed under reduced pressure, and the residue was added to water (100 ml) and extracted with dichloromethane (100 ml). The extract was concentrated under reduced pressure. Infra-red spectroscopic examination of the residue indicated the absence of nitrile groups. The product was concluded to be substantially 2,2'-azobis(n-butyl 2-methylpropanoate), having the formula:-

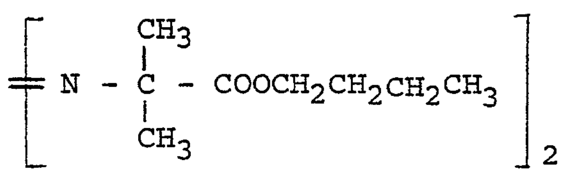

COMPARATIVE EXAMPLE

The procedure of Example 5 was repeated, but omitting the 2-ethoxyethyl acetate. Infra-red spectroscopic examination of the product indicated that only about 40% by weight of it was the azoester desired.

EXAMPLE 6

Octanol (26 g, 0 2 mol) and 2,2'-azobis(2-methylpropionitrile)(16.4 g, 0.1 mol) were added to diethylether (100 ml) and the mixture was stirred. Dry hydrogen chloride gas was bubbled into the cooled reaction mixture until it was saturated. After 2 days at room temperature the reaction mixture was a colourless immobile slurry and infra-red spectroscopic examination indicated the absence of any nitrile group absorption. Solvents were removed under reduced pressure and the resulting crystalline solid added to saturated aqueous sodium bicarbonate solution (500 ml). The product was extracted into dichloromethane (4 x 100 ml); the combined organic extracts were washed with water (3 x 200 ml), dried (anhydrous magnesium sulphate) and concentrated under reduced pressure to give a mobile yellow liquid (23.2 g). When examined by infra-red spectroscopy, the product exhibited, in addition to the ester peak at 1735 $cm^{-1}$, a substantial peak at 1650 $cm^{-1}$ characteristic of the imidate grouping. It was therefore concluded that the product contained not only the expected ester, 2,2'-azobis(octyl 2-methylpropanoate), but also unhydrolysed iminoether.

WHAT WE CLAIM IS:-

1. A process for the preparation of azoiminoether hydrochlorides of the general formula

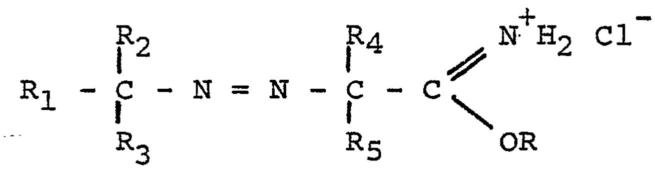

(I)

wherein R is the residue of a monohydroxy compound, $R_1$ is a methyl or ethyl group or a grouping of the formula

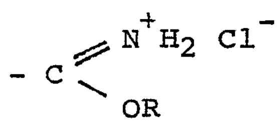

; $R_2$ is a methyl group or a β-carboxymethyl group and $R_3$ is a methyl group, or $R_2$ and $R_3$ together with the intermediate carbon atom form a cyclohexyl group; $R_4$ is a methyl group and $R_5$ is a methyl group, an ethyl group, a β-carboxyethyl group or a β-methoxy-ββ-dimethylethyl group, or $R_4$ and $R_5$ together with the intermediate carbon atom form a cyclohexyl group, the process comprising the reaction of an azonitrile of the formula

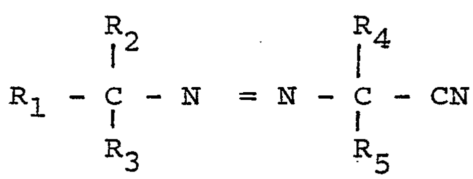

(II)

wherein $R_1$ is a methyl or ethyl group or a -CN group, $R_2$, $R_3$, $R_4$ and $R_5$ have the same significance as in formula (I), with a stoichiometrically equivalent amount of a monohydroxy compound R-OH in the presence of hydrogen chloride gas and of a compound containing in the molecule at least one ether grouping -C-O-C- , the reaction being conducted under anhydrous conditions.

2. A process as claimed in claim 1, wherein the azonitrile is 2,2'-azobis(2-methylpropionitrile).

3. A process as claimed in claim 1 or claim 2, wherein the monohydroxy compound is a lower aliphatic monohydric alcohol.

4. A process as claimed in any one of claims 1 to 3, wherein the ether group-containing compound is diethyl ether, dioxan, 2-ethoxyethyl acetate or diethylene glycol dimethyl ether.

5. A process as claimed in any one of claims 1 to 4, wherein the proportion used of the ether group-containing compound is sufficient to provide at least two ether oxygen atoms for each nitrile group present.

6. A process as claimed in any one of claims 1 to 5, wherein the reaction mixture contains an inert liquid diluent or solvent.

7. A process as claimed in claim 1, wherein the monohydroxy compound and the ether group-containing compound are constituted by a single substance containing both types of function in the molecule.

8. A process as claimed in claim 7, wherein the single substance is a monohydroxypoly(alkyleneoxy) compound.

9. A process as claimed in claim 8, wherein the monohydroxypoly(alkyleneoxy) compound is the monomethyl ether of a polyethylene glycol of molecular weight 250-4000.

10. A process according to claim 1 substantially as hereinbefore described with reference to the foregoing Examples.

11. An azoiminoether hydrochloride of the general formula (I) whenever prepared by a process as claimed in any one of claims 1 to 10.

12. A process for the preparation of an azocarboxylic ester which comprises the hydrolysis of an azoiminoether hydrochloride obtained according to any one of claims 1 to 10.

0080275

European Patent Office

# EUROPEAN SEARCH REPORT

Application number

EP 82 30 5791

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | CHEMICAL REVIEWS, no.61, 1961 R. ROGER et al.: "The chemistry of imidates", pages 179-211 * page 181 III.A.1, lines 11-15 * | 1-6 | C 07 C 107/02<br>C 08 F 4/04 |
| X | DE-A-2 254 572 (BAYER AG.) * page 1, line 15 - page 2, line 24; examples * | 1-6,11 | |
| X | DE-A-2 124 000 (MERCK PATENT GmbH) * claims; examples 6-8,10,11 * | 1-11 | |
| X | US-A-4 290 945 (A.A. SYROV) * example 14; claims * | 1-11 | |

TECHNICAL FIELDS SEARCHED (Int. Cl. 3)

C 07 C 107/00

The present search report has been drawn up for all claims

| Place of search | Date of completion of the search | Examiner |
|---|---|---|
| THE HAGUE | 14-02-1983 | PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82